# EUROPEAN PATENT APPLICATION

(11) **EP 1 859 808 A1**
(43) Date of publication of application: **28.11.2007**
(21) Application number: 06729043.7
(22) Date of filing: 14.03.2006
(51) Int. Cl.: A61K 45/00, A61K 31/7088, A61K 48/00, A61P 3/08, C12N 1/15, C12N 1/19, C12N 1/21, C12N 5/00, C12N 15/09

(54) **NOVEL SUGAR UPTAKE ACTIVATOR AND METHOD FOR SCREENING THE SAME**

(30) Priority: 15.03.2005 JP 2005072457
(71) Applicant: Astellas Pharma Inc., Tokyo 103-8411 (JP)
(72) Inventor: GOTO, Masahide, 2-chome, Chuo-ku, Tokyo, 1038411 (JP); SHIMOKAWA, Teruhiko, 2-chome, Chuo-ku, Tokyo, 1038411 (JP)
(74) Representative: HOFFMANN EITLE
(86) International application number: PCT/JP2006/305009
(87) International publication number: WO 2006/098314

(57) **Abstract**

An activating agent of glucose uptake comprising as an active ingredient a substance having a PGC-1β function; and a method of screening for an activating agent of glucose uptake, comprising the steps of bringing a substance to be tested into contact with a promoter of a PGC-1β gene and analyzing a promoter activity of a PGC-1β gene, are disclosed. The activating agent of glucose uptake is a novel antidiabetic agent capable of promoting glucose uptake in a muscle tissue at hyperglycemia regardless of a blood insulin level.

SEQUENCE LISTING

<110> Astellas Pharma Inc.

<120> A novel activating agent of glucose uptake and a screening method
therefor

<130> AP05079-754

<150> JP 2005-72457
<151> 2005-03-15

<160> 11

<170> PatentIn version 3.1

<210> 1
<211> 1028
<212> DNA
<213> Homo sapiens

<220>
<223> Inventor: Goto, Masahide; Shimokawa, Teruhiko

<400> 1
gaatataggc aaacgcaccc ttcaagtaaa agataaaaat gcctcctcct ccaaggagac 60

ttccttgatt tctttgcttt tgatctatgg tatttctccc tccttctctg gccttgtagc 120

ctgggtagag aaacagtcct ccaggggagt aagacctaag ctttcccttc ctgcaactga 180

tgtgcgctgt agctttgaac gaggtcctag gtatatacat acatacatac atacatacat 240

acatacatat atatatacat acatatatat acacatacat atatatacat acatatatat 300

acacatacat acatacatat atacacatac atacatacat acatatatat acacatacat 360

acatatatat atatatatat attttttttt tttttttttt ttcaggaggg atatgaagag 420

tgacttacag gcgcccgcca ccatgcctgg ctaatttttt ttgtattttc agtagagacg 480

gggtttcacc atgttggcca ggctggtctc aaactcctga cctcaggtga tccacccgcc 540

tcggcctccc aaagtgctgg gattacaggc gtgagccact gcgcccggcc ttcgaacgag 600

gtcctattcc ttctcgagcc tcagtttccc cagctgtgca gaagaagcag gttgtctcgc 660

aggacccgcc cagcgctaac gcaacgtgat tcgctctggc aggagccggg ggctgcccgc 720

attgctgcgc aggcatcggg ctgctggctg gggcaccgcc acctggctgc ctgcgcttta 780

ccccttgctg agggctgcgt gagctagtcg cggcgccaga cacggcgcag gaaagtgggt 840

gagcgacccc cggctcccgc gggcgccgcg cggccccgcc cccgcagcta gcggccctgc 900

ggcagccggg ggctcgagct ccgccctccg cctcccgccg gcctcactcc ctcctccctc 960

ctcccttgct cgctcgctgg ctccctcccc ccgggccggc tcggcgttga ctccgccgca 1020

cgcttcag 1028

<210> 2
<211> 25
<212> DNA
<213> Homo sapiens

<400> 2
acgctggtac cgcggctgga agatg 25

<210> 3
<211> 29
<212> DNA
<213> Homo sapiens

<400> 3
tcgagggcgg ccgctgttat caatgcagg 29

<210> 4
<211> 20
<212> DNA
<213> Rattus sp.

<400> 4
tactgccctg gctcctagca 20

<210> 5
<211> 23
<212> DNA
<213> Rattus sp.

<400> 5
tcaggaggag caatgatctt gat 23

<210>  6
<211>  24
<212>  DNA
<213>  Rattus sp.

<400>  6
tgctttccta ctatgactcc tccc                                              24


<210>  7
<211>  22
<212>  DNA
<213>  Rattus sp.

<400>  7
aacccccttc acccagagtc ta                                                22


<210>  8
<211>  29
<212>  DNA
<213>  Homo sapiens

<400>  8
caggtttggt accgaatata ggcaaacgc                                         29


<210>  9
<211>  30
<212>  DNA
<213>  Homo sapiens

<400>  9
ttccagacgc gtctgcagcg tgcggcggag                                        30


<210>  10
<211>  3072
<212>  DNA
<213>  Homo sapiens

<220>
<221>  CDS
<222>  (1)..(3072)
<223>

<400> 10
   atg gcg ggg aac gac tgc ggc gcg ctg ctg gac gaa gag ctc tcc tcc 48
   ttc ttc ctc aac tat ctc gct gac acg cag ggt gga ggg tcc ggg gag 96
   gag caa ctc tat gct gac ttt cca gaa ctt gac ctc tcc cag ctg gat 144
   gcc agc gac ttt gac tcg gcc acc tgc ttt ggg gag ctg cag tgg tgc 192
   cca gag aac tca gag act gaa ccc aac cag tac agc ccc gat gac tcc 240
   gag ctc ttc cag att gac agt gag aat gag gcc ctc ctg gca gag ctc 288
   acc aag acc ctg gat gac atc cct gaa gat gac gtg ggt ctg gct gcc 336
   ttc cca gcc ctg gat ggt gga gac gct cta tca tgc acc tca gct tcg 384
   cct gcc ccc tca tct gca ccc ccc agc cct gcc ccg gag aag ccc tcg 432
   gcc cca gcc cct gag gtg gac gag ctc tca ctg ctg cag aag ctc ctc 480
   ctg gcc aca tcc tac cca aca tca agc tct gac acc cag aag gaa ggg 528
   acc gcc tgg cgc cag gca ggc ctc aga tct aaa agt caa cgg cct tgt 576
   gtt aag gcg gac agc acc caa gac aag aag gct ccc atg atg cag tct     624
   cag agc cga agt tgt aca gaa cta cat aag cac ctc acc tcg gca cag     672
   tgc tgc ctg cag gat cgg ggt ctg cag cca cca tgc ctc cag agt ccc     720
   cgg ctc cct gcc aag gag gac aag gag ccg ggt gag gac tgc ccg agc     768
   ccc cag cca gct cca gcc tct ccc cgg gac tcc cta gct ctg ggc agg     816
   gca gac ccc ggt gcc ccg gtt tcc cag gaa gac atg cag gcg atg gtg     864
   caa ctc ata cgc tac atg cac acc tac tgc ctc ccc cag agg aag ctg     912
   ccc cca cag acc cct gag cca ctc ccc aag gcc tgc agc aac ccc tcc     960
   cag cag gtc aga tcc cgg ccc tgg tcc cgg cac cac tcc aaa gcc tcc     1008
   tgg gct gag ttc tcc att ctg agg gaa ctt ctg gct caa gac gtg ctc     1056
   tgt gat gtc agc aaa ccc tac cgt ctg gcc acg cct gtt tat gcc tcc     1104
   ctc aca cct cgg tca agg ccc agg ccc ccc aaa gac agt cag gcc tcc     1152
   cct ggt cgc ccg tcc tcg gtg gag gag gta agg atc gca gct tca ccc      1200
   aag agc acc ggg ccc aga cca agc ctg cgc cca ctg cgg ctg gag gtg      1248
   aaa agg gag gtc cgc cgg cct gcc aga ctg cag cag cag gag gag gaa      1296
   gac gag gaa gaa gag gag gag gaa gag gaa gaa gaa aaa gag gag gag      1344
   gag gag tgg ggc agg aaa agg cca ggc cga ggc ctg cca tgg acg aag      1392
   ctg ggg agg aag ctg gag agc tct gtg tgc ccc gtg cgg cgt tct cgg      1440
   aga ctg aac cct gag ctg ggc ccc tgg ctg aca ttt gca gat gag ccg      1488
   ctg gtc ccc tcg gag ccc caa ggt gct ctg ccc tca ctg tgc ctg gct      1536
   ccc aag gcc tac gac gta gag cgg gag ctg ggc agc ccc acg gac gag      1584
   gac agt ggc caa gac cag cag ctc cta cgg gga ccc cag atc cct gcc      1632
   ctg gag agc ccc tgt gag agt ggg tgt ggg gac atg gat ggg gac ccc      1680
   agc tgc ccg cag ctc cct ccc aga gac tct ccc agg tgc ctc atg ctg      1728
   gcc ttg tca caa agc gac cca act ttt ggc aag aag agc ttt gag cag 1776
   acc ttg aca gtg gag ctc tgt ggc aca gca gga ctc acc cca ccc acc 1824
   aca cca ccg tac aag ccc aca gag gag gat ccc ttc aaa cca gac atc 1872
   aag cat agt cta ggc aaa gaa ata gct ctc agc ctc ccc tcc cct gag 1920
   ggc ctc tca ctc aag gcc acc cca ggg gct gcc cac aag ctg cca aag 1968
   aag cac cca gag cga agt gag ctc ctg tcc cac ttg cga cat gcc aca 2016
   gcc cag cca gcc tcc cag gct ggc cag aag cgt ccc ttc tcc tgt tcc 2064
   ttt gga gac cat gac tac tgc cag gtg ctc cga cca gaa ggc gtc ctg 2112
   caa agg aag gtg ctg agg tcc tgg gag ccg tct ggg gtt cac ctt gag 2160
   gac tgg ccc cag cag ggt gcc cct tgg gct gag gca cag gcc cct ggc 2208
   agg gag gaa gac aga agc tgt gat gct ggc gcc cca ccc aag gac agc 2256
   acg ctg ctg aga gac cat gag atc cgt gcc agc ctc acc aaa cac ttt 2304
   ggg ctg ctg gag acc gcc ctg gag gag gaa gac ctg gcc tcc tgc aag 2352
   agc cct gag tat gac act gtc ttt gaa gac agc agc agc agc agc ggc 2400
   gag agc agc ttc ctc cca gag gag gaa gag gaa gaa ggg gag gag gag 2448
   gag gag gac gat gaa gaa gag gac tca ggg gtc agc ccc act tgc tct 2496
   gac cac tgc ccc tac cag agc cca cca agc aag gcc aac cgg cag ctc 2544
   tgt tcc cgc agc cgc tca agc tct ggc tct tca ccc tgc cac tcc tgg 2592
   tca cca gcc act cga agg aac ttc aga tgc gag agc aga ggg ccg tgt 2640
   tca gac aga acg cca agc atc cgg cac gcc agg aag cgg cgg gaa aag 2688
   gcc att ggg gaa ggc cgc gtg gtg tac att caa aat ctc tcc agc gac 2736
   atg agc tcc cga gag ctg aag agg cgc ttt gaa gtg ttt ggt gag att 2784
   gag gag tgc gag gtg ctg aca aga aat agg aga ggc gag aag tac ggc 2832
   ttc atc acc tac cgg tgt tct gag cac gcg gcc ctc tct ttg aca aag 2880
   ggc gct gcc ctg agg aag cgc aac gag ccc tcc ttc cag ctg agc tac      2928
   gga ggg ctc cgg cac ttc tgc tgg ccc aga tac act gac tac gat tcc      2976
   aat tca gaa gag gcc ctt cct gcg tca ggg aaa agc aag tat gaa gcc      3024
   atg gat ttt gac agc tta ctg aaa gag gcc cag cag agc ctg cat      3069
   tga                                                                  3072

<210> 11
<211> 1023
<212> PRT
<213> Homo sapiens

<400> 11

## Description

### TECHNICAL FIELD

The present invention relates to an activating agent of glucose uptake comprising as an active ingredient a substance having a PGC-1β function, a method of screening for an activating agent of glucose uptake using a polynucleotide having a promoter activity of a PGC-1β gene, and a method of analyzing an activity of glucose uptake.

### BACKGROUND ART

Diabetes is a disease characterized by chronic hyperglycemia caused by a deficiency of insulin action, and classified by cause into two types: type 1 diabetes caused by an absolute deficiency of insulin, and type 2 diabetes caused by a deficiency of insulin action (non-patent reference 1). Diabetes progresses without subjective symptoms for a long time, and during that time, microangiopathy progresses and complications associated with diabetes, such as retinopathy, nephropathy; or neuropathy, develop. Further, diabetes is known as an important risk factor for arteriosclerosis including, for example, cerebral infarction or ischemic heart disease such as cardiac infarction or angina pectoris, and thus, developments in effective therapeutic agents or treatments for diabetes are required.

Type 1 diabetes and type 2 diabetes as classified by cause account for approximately 10% and 90% of patients suffering from diabetes, respectively, and type 2 shows an extremely high incidence rate. Type 2 diabetes is also called Non-Insulin Dependent Diabetes Mellitus (NIDDM), which shows hyperglycemia regardless of blood insulin level. It is recognized that type 2 diabetes is caused by a decreased sensitivity of organs to insulin. The decreased sensitivity to insulin causes an increase in the blood insulin level needed to maintain a normal blood glucose level in a living body, and results in a state called "insulin resistance" (non-patent reference 2). Obesity is suggested as a factor for the insulin resistance, and among various types of obesity, obesity accompanied by an overaccumulation and increase of visceral fat (visceral obesity) has become known as a risk factor for diabetes (non-patent reference 2). Under these circumstances, the development of an agent capable of increasing the sensitivity of organs to insulin (an agent for alleviating insulin resistance) or an agent for alleviating visceral obesity, which causes insulin resistance, is desired, but an agent which has a satisfactory major drug effect and no adverse effects has not been found.

Recently, it was found from studies of obesity that a nuclear receptor family member, peroxisome proliferator-activated receptor γ (PPARγ), plays an important role in adipogenesis (non-patent reference 4). Further, it was found that thiazolidinedione derivatives (TZD derivatives) developed as a hypoglycemic agent have an agonist activity capable of activating PPARγ, and the TZD derivatives are clinically used as a therapeutic agent. However, with respect to the TZD derivatives, a risk of heart failure or edema caused by a systemic fluid retention, an adverse effect such as a promotion of obesity associated with an increase or enlargement of adipocytes, and a problem of the existence of nonresponders not affected thereby have been reported (FDA prescribing information), and thus, the TZD derivatives are not entirely satisfactory as an agent for alleviating insulin resistance in type 2 diabetes. Developments in novel agents without these adverse effects are greatly desired.

It was clarified from biochemical and molecular biological studies in PPARγ that, in addition to a regulation of the PPARγ activity by a physiological agonist instead of the TZD derivatives, other regulations thereof by a transcriptional coactivator(s) and a transcriptional corepressor(s) are important to express the function of PPARγ (non-patent reference 5). PPARγ coactivator-1α (PGC-1α), which is considered to be one of such regulatory proteins, was reported to have a function as a coactivator capable of strongly inducing the transcriptional activity of PPARγ by interaction with PPARγ (non-patent reference 6). It was clarified from a functional analysis of PGC-1α that PGC-1α functions as a coactivator of various nuclear receptors, such as PPARα, PPARδ, thyroid receptor α (TRα), or estrogen receptor α (ERα), as well as PPARγ, and regulates or controls the gene expression levels of various molecules including glucose transporters and mitochondrial proteins involved in ATP synthesis or thermogenesis; a function which activates a promotion of energy metabolism by a combustion of sugars or fats, due to an increase in oxygen consumption or an increase in the number of mitochondria, was physiologically expected; and it was suggested that PGC-1α would be useful for the treatment of type 2 diabetes or obesity (non-patent reference 7). However, it was found from subsequent studies that PGC-1α functions in the liver as a coactivator of a transcriptional factor, hepatocyte nuclear factor 4α (HNF4α) and, as a result, PGC-1α induces expressions of phosphoenolpyruvate carboxykinase (PEPCK) and glucose-6-phosphatase as a rate limiting enzyme in gluconeogenesis, and promotes the gluconeogenesis in the liver; there is concern therefore that there is a possibility that an increase in or activation of PGC-1α will complicate diabetes (non-patent reference 8).

Recently, PGC-1β was reported as a molecule different from PGC-1α (non-patent reference 9). Namely, it was reported that PGC-1β binds to a nuclear receptor ERR (Estrogen Receptor-related Receptor) in a relatively selective manner to induce an expression of medium chain acyl-CoA dehydrogenase (MCAD) known as a rate limiting enzyme in β oxidation of fatty acids under the expression control of ERR; that PGC-1β has an activity of promoting energy metabolism caused by an increase in the number of mitochondria in muscle cells; and that PGC-1β plays a physiological role different from PGC-1α (non-patent reference 10). Further, it was reported that transgenic mice systemically overexpressing PGC-1β were made to lose weight by a promotion of energy metabolism, and showed a decrease in an amount of adipose tissues and decreases in concentrations of cholesterol, insulin, and leptin in blood (non-patent reference 11). Furthermore, it was suggested that PGC-1β would not affect gluconeogenesis in the liver, because PGC-1β did not promote an expression of rate limiting enzymes in gluconeogenesis in hepatocytes whereas PGC-1α did (non-patent reference 12). However, the relationship between PGC-1β and glucose uptake is not known.

Although a nucleotide sequence of a coding region of a human PGC-1β gene was first disclosed in patent reference 1, a DNA having a PGC-1β promoter activity has not yet been obtained, and an assay system suitable as a screen for a substance capable of promoting a PGC-1β expression has not been established.

[non-patent reference 1] Japan Diabetes Society, Tounyoubyou chiryou gaido 2000-2003 (Treatment of diabetes mellitus, Guide 2000-2003), Bunkodo, 2002, p.6-11
[non-patent reference 2] Yukimasa HIRATA, Tounyoubyou no chiryou (Treatment of diabetes), 2nd ed., Bunkodo, 2003, p.821-907
[non-patent reference 3] Metabolism, U.S.A., 1987, vol.36, p.54-59
[non-patent reference 4] Cell, U.S.A., 1994, vol.79, p.1147-1156
[non-patent reference 5] Nature, United Kingdom, 1998, vol.395, p.137-143
[non-patent reference 6] Cell, U.S.A., 1998, vol.92, p.829-838
[non-patent reference 7] Cell, U.S.A., 1999, vol.98, p.115-124
[non-patent reference 8] Nature, United Kingdom, 1998, vol.413, p.131-138
[non-patent reference 9] The Journal of Biological Chemistry, U.S.A., 2002, vol.277, p.1645-1648
[non-patent reference 10] The Journal of Biological Chemistry, U.S.A., 2003, vol.278, p.26597-26603
[non-patent reference 11] Proceedings of the National Academy of Sciences of the United States of America, U.S.A., 2003, vol.100, p.12378-12383
[non-patent reference 12] The Journal of Biological Chemistry, U.S.A., 2003, vol.278, p.30843-30848
[patent reference 1] International Publication No. WO 02/22818

### DISCLOSURE OF INVENTION

### PROBLEMS TO BE SOLVED BY THE INVENTION

An object of the present invention is to provide an activating agent of glucose uptake, which is a novel antidiabetic agent capable of promoting glucose uptake in a muscle tissue at hyperglycemia regardless of a blood insulin level, and a method of screening for the activating agent of glucose uptake.

### MEANS FOR SOLVING THE PROBLEMS

PGC-1β activates mitochondrial activities, and thus, an antiobesity effect on the basis of the activation of intracellular energy metabolism was expected. Further, it was suggested that PGC-1β would alleviate insulin resistance and exhibit a therapeutic effect on diabetes, as a result of the alleviation of obesity, a risk factor for diabetes. The present inventors conducted intensive studies and, as a result, found that PGC-1β unexpectedly exhibits an activity of promoting glucose uptake in a muscle tissue regardless of the insulin level, and completed providing a method of screening for an activating agent of glucose uptake, and an activating agent of glucose uptake. As described above, the activating agent of glucose uptake based on the activation of PGC-1β is quite different from known agents, such as a PPAR_{Y} agonist, for alleviating insulin resistance on the basis of an activity of promoting obesity. Further, the activating agent of glucose uptake is not an agent for alleviating insulin resistance on the basis of only an antiobesity activity, but is one which promotes glucose uptake into organs insulin affects, regardless of the insulin level. Furthermore, the activation of PGC-1β is different from that of PGC-1α in that PGC-1β does not exhibit a gluconeogenetic activity in the liver, and promotes the glucose uptake even in the absence of insulin. It is considered from these findings that the activating agent of the present invention would be a novel medicament for patients who do not exactly coincide with patients alleviated by known agents for alleviating insulin resistance, and has a possibility of fulfilling such unmet needs.

The present inventors conducted intensive studies into solving the above object and, as a result, found that muscle cell line L6 overexpressing human PGC-1β, using a recombinant adenovirus of human PGC-1β, induced a gene expression of an intracellular glucose transporter GLUT4 (Glucose Transporter 4) (Example 5) and promoted an activity of glucose uptake in the cells (Example 6). The present inventors found that PGC-1β promoted glucose uptake much more highly than PGC-1α, and showed a remarkable effect in that PGC-1β promoted glucose uptake even in the absence of insulin (Example 6). From a human genomic DNA, the present inventors isolated a promoter region consisting of a sequence of approximately 1 kb upstream of a PGC-1β gene, and established a screening method for a substance which regulates the promoter activity, using the isolated promoter region (Examples 7 to 10). The present inventors obtained a substance capable of activating the promoter by using the screening method (Example 11), and confirmed that the obtained substance induced an activity of glucose uptake in muscle cells (Example 12), and thus, the present invention was completed.

The present invention relates to:
[1] an activating agent of glucose uptake comprising as an active ingredient a substance having a PGC-1β function;
[2] a method for activating glucose uptake, comprising administering to a subject in need thereof a substance having a PGC-1β function, in an amount effective therefor;
[3] Use of a substance having a PGC-1β function in the manufacture of an activating agent of glucose uptake;
[4] a polynucleotide selected from the group consisting of:
   a polynucleotide consisting of a nucleotide sequence in which 1 to 10 nucleotides are deleted, substituted, and/or added in the nucleotide sequence of SEQ ID NO: 1 or a partial sequence thereof, and having a promoter activity of a PGC-1β gene, and
   a polynucleotide consisting of the nucleotide sequence of SEQ ID NO: 1 or a partial sequence thereof;
[5] the polynucleotide of [4], consisting of a nucleotide sequence in which 1 to 10 nucleotides are deleted, substituted, and/or added in the nucleotide sequence of SEQ ID NO: 1, and having a promoter activity of a PGC-1β gene, or consisting of the nucleotide sequence of SEQ ID NO: 1;
[6] use of the polynucleotide of [4] as a promoter of a gene;
[7] a recombinant vector, comprising the polynucleotide of [4];
[8] a transformant comprising the polynucleotide of [4];
[9] use of the polynucleotide of [4] as a screening tool for an activating agent of glucose uptake;
[10] use of the polynucleotide of [4] in the screening for an activating agent of glucose uptake;
[11] a method of screening for an activating agent of glucose uptake, comprising the steps of:
   bringing a substance to be tested into contact with a polynucleotide comprising the polynucleotide of [4], and
   analyzing a promoter activity of a PGC-1β gene;
[12] the method of [11], wherein the polynucleotide comprising the polynucleotide of [4] contains a reporter gene at the downstream region thereof, and the step of analyzing a promoter activity of a PGC-1β gene is a step of analyzing an expression of the reporter gene; and
[13] a method of analyzing an activity of glucose uptake, comprising the steps of:
   bringing a substance to be tested into contact with the polynucleotide comprising the polynucleotide of [4], analyzing a promoter activity of a PGC-1β gene, and
   analyzing an activity of glucose uptake caused by the test substance.

The nucleotide sequence consisting of nucleotides 1-1028 of SEQ ID NO: 1 corresponds to -996 to +32 of the human PGC-1β gene. The screening method of the present invention preferably comprises a step of selecting a substance which promotes the promoter activity of a PGC-1β gene, and a luciferase gene is preferable as "the reporter gene." "The expression of the reporter gene" can be confirmed by analyzing (particularly measuring) an activity (reporter activity) of a polypeptide encoded by the gene.

The term, substance having a PGC-1β function, as used herein means PGC-1β, a polynucleotide encoding PGC-1β, a substance which increases an amount of PGC-1β expressed, or a substance having an activity of activating a promoter of a PGC-1β gene. An activating agent of glucose uptake, selected by the screening method of the present invention, exhibits an activation of a human PGC-1β promoter.

The term, activation of glucose uptake, as used herein means an increase in the number of glucose molecules incorporated into a cell.
The term, analysis, as used herein includes a detection to judge a presence or absence of a substance to be analyzed, and a measurement to quantitatively or semi-quantitatively determine an amount of a substance to be analyzed.

### EFFECTS OF THE INVENTION

According to the screening method of the present invention, an activating agent of glucose uptake, which is a novel antidiabetic agent capable of promoting glucose uptake in a muscle tissue at hyperglycemia regardless of a blood insulin level, can be provided. The activating agent of glucose uptake according to the present invention, or an activating agent of glucose uptake obtained by the screening method of the present invention is not an agent for alleviating insulin resistance on the basis of only an antiobesity activity, but is one which promotes glucose uptake into organs insulin affects, regardless of the insulin level, and will fulfill unmet needs of patients not alleviated by known agents for alleviating insulin resistance.

### BRIEF DESCRIPTION OF THE DRAWINGS

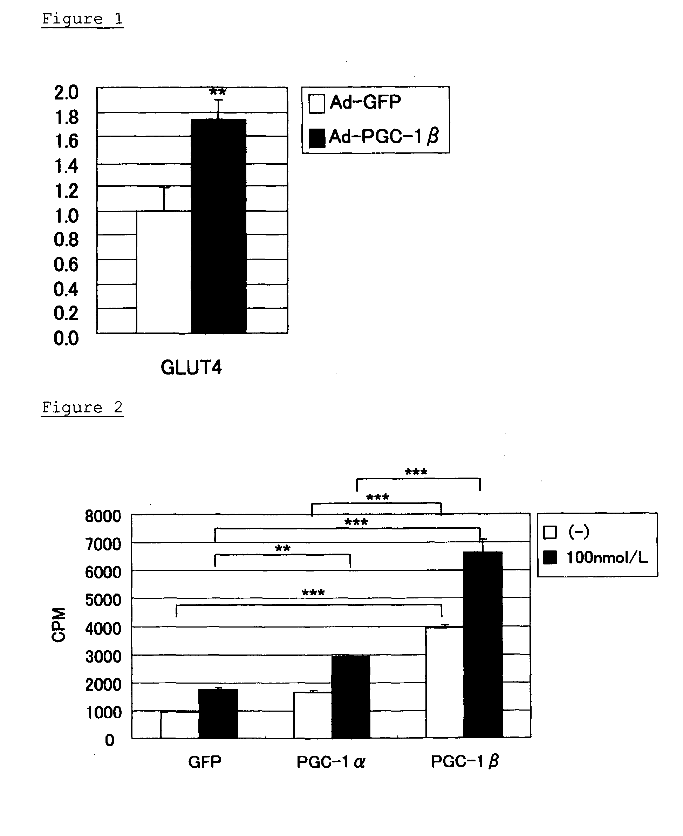

Figure 1 is a graph showing the result of an induction of GLUT4 gene expression in muscle cells overexpressing human PGC-1β. The vertical axis indicates a relative amount of gene expressed, and the symbol ** denotes that a significant difference was p<0.01 (Dunnett test).
Figure 2 is a graph showing the result of measuring an activity of glucose uptake in muscle cells overexpressing human PGC-1β, in comparison with PGC-1α. The value described in the right box indicates an insulin concentration. The symbols ** and *** denote that significant differences were p<0.01 and p<0.001 (Dunnett test), respectively.

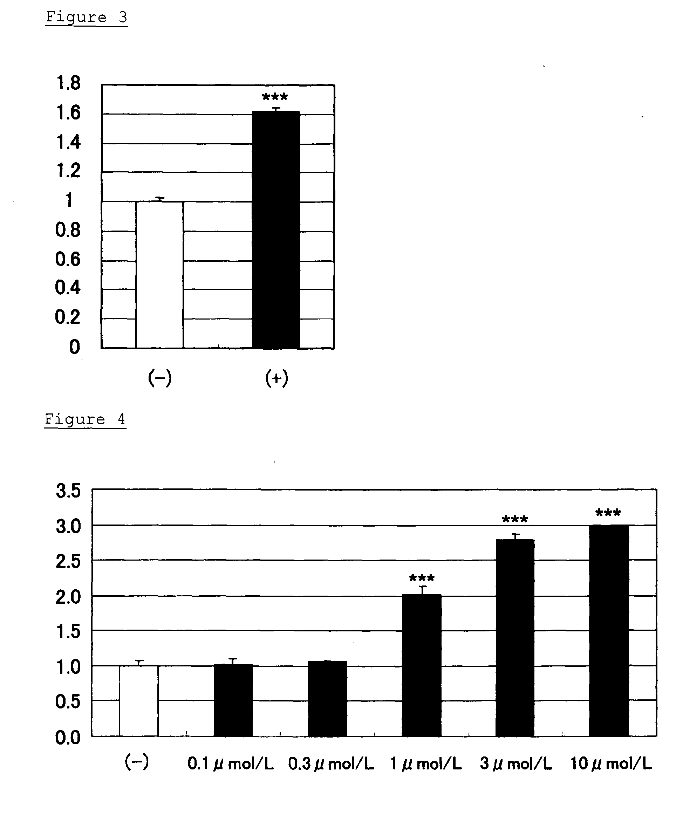

Figure 3 is a graph showing the result of measuring an activity of human PGC-1β promoter. The vertical axis indicates a relative activity, and the symbol *** denotes that a significant difference was p<0.001 (Dunnett test). The symbol (+) indicates the result when forskolin and dexamethasone were added, and the symbol (-) indicates the result when neither forskolin nor dexamethasone were added (i.e., DMSO as a solvent was added alone).
Figure 4 is a graph showing the result of screening for an activating agent of a human PGC-1β promoter. The vertical axis indicates a relative activity, and the horizontal axis indicates a concentration of compound A. The symbol *** denotes that a significant difference was p<0.001 (Dunnett test).

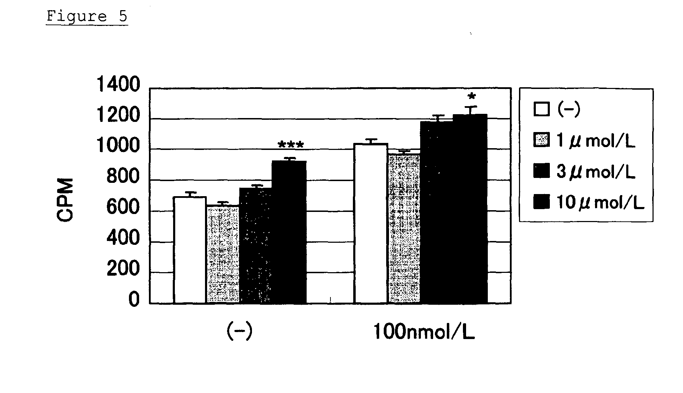

Figure 5 is a graph showing the result of measuring an activity of compound A on glucose uptake. The horizontal axis indicates an insulin concentration, and each value described in the right box indicates a concentration of compound A. The symbols * and *** denote that significant differences were p<0.05 and p<0.001 (Dunnett test), respectively.

### BEST MODE FOR CARRYING OUT THE INVENTION

The present invention will be explained in detail hereinafter.

### 1. Activating agent of glucose uptake comprising as an active ingredient a substance having a PGC-1β function

The present invention includes an activating agent of glucose uptake comprising as an active ingredient a substance having a PGC-1β function, that is, PGC-1β, a polynucleotide encoding PGC-1β, a substance which increases an amount of PGC-1β expressed, or a substance having an activity to induce a promoter of a PGC-1β gene. The present invention includes a method for activating glucose uptake, comprising administering to a subject in need thereof a substance having a PGC-1β function (PGC-1β, a polynucleotide encoding PGC-1β, a substance which increases an amount of PGC-1β expressed, or a substance having an activity of activating a promoter of a PGC-1β gene), in an amount effective therefor. The present invention includes use of a substance having a PGC-1β function (PGC-1β, a polynucleotide encoding PGC-1β, a substance which increases an amount of PGC-1β expressed, or a substance having an activity of activating a promoter of a PGC-1β gene) in the manufacture of an activating agent of glucose uptake.

As the PGC-1β, naturally-occurring PGC-1β may be used as an active ingredient of the activating agent of glucose uptake according to the present invention.

As the PGC-1β,
(1)a polypeptide comprising an amino acid sequence having a 90% or more identity, preferably a 95% or more identity, with human PGC-1β consisting of the amino acid sequence of SEQ ID NO: 11, and exhibiting a PGC-1β activity (hereinafter referred to as a homologous polypeptide); or
(2) a polypeptide comprising an amino acid sequence in which one or several (for example, 1 to 10) amino acids are deleted, substituted, and/or added in the amino acid sequence of SEQ ID NO: 11, and exhibiting a PGC-1β activity (hereinafter referred to as a variation functionally equivalent)
   is more preferable, and human PGC-1β consisting of the amino acid sequence of SEQ ID NO: 11 is most preferable, independently of being identical to naturally-occurring sequences.
   The term, PGC-1β activity, as used herein means an activity of activating (i.e., promoting or inducing) glucose uptake, that is, an activity of increasing an amount of glucose molecules incorporated into a cell.

The term, identity, as used herein means a value obtained by a BLAST (Basic local alignment search tool; Altschul, S. F. et al., J. Mol. Biol., 215, 403-410, 1990). The homology in the amino acid sequence may be calculated by a BLAST search algorithm. More particularly, it may be calculated using a b12seq program (Tatiana A. Tatusova and Thomas L. Madden, FEMS Microbiol. Lett., 174, 247-250, 1999) in a BLAST package (sgi32bit edition, version 2.0.12; obtained from NCBI) in accordance with a default parameter. As a pairwise alignment parameter, a program blastp is used. Further, 0 as a Gap insertion cost value, 0 as a Gap elongation cost value, SEG as a filter for a Query sequence, and BLOSUM62 as a Matrix are used, respectively.

The polynucleotide encoding PGC-1β is not particularly limited, so long as it encodes PGC-1β. As the polynucleotide, a polynucleotide encoding the homologous polypeptide or the variation functionally equivalent is preferable, and a polynucleotide encoding human PGC-1β (most preferably, a polynucleotide consisting of the nucleotide sequence of SEQ ID NO: 10) is more preferable.

Whether or not a certain polypeptide (hereinafter referred to as a test polypeptide) exhibits the PGC-1β activity, that is, whether or not a test polypeptide exhibits an activity of activating glucose uptake, may be confirmed by a conventional method known to those skilled in the art, or a modification thereof, such as a method described in Example 6. More particularly, appropriate cells (preferably muscle cells) transformed with an expression vector capable of expressing the test polypeptide may be cultivated for a predetermined time (for example, 10 minutes) in the presence of labeled glucose, and an amount of glucose incorporated into the cells may be measured by using the label as an index, to confirm the activity of activating glucose uptake.

Whether or not a substance to be tested exhibits an activity of activating a promoter of a PGC-1β gene may be confirmed in accordance with the "step of bringing a substance to be tested into contact with a polynucleotide for screening" and the "step of analyzing a promoter activity of a PGC-1β gene" in "3. Screening method of the present invention" described below. When a method described in Example 11 is used for the confirmation, a substance in which the activity of activating the promoter is 2 times or more, in comparison with a control case in the absence of the substance, is most preferable.

The activating agent of glucose uptake comprising as an active ingredient a substance having a PGC-1β function may be composed of the active ingredient alone or alternatively, may be prepared using carriers, fillers, and/or other additives generally used in the preparation of medicaments, if desired, in accordance with the active ingredient.

Examples of administration include oral administration by tablets, pills, capsules, granules, fine granules, powders, oral solutions and the like, and parenteral administration by injections (e.g., intravenous, intramuscular, or the like), suppositories, transdermal preparations, transmucosal absorption preparations and the like. Particularly, in the case of polypeptides which are digested by enzymes, a parenteral administration such as a transdermal preparation or intravenous injection or the like, or an administration using formulations in which the polypeptide is delivered without digestion to a lower gastrointestinal tract (such as jejunum, ileum, colon, or large intestine) where digestive enzymes are not very effective, is desirable.

In the solid composition for use in the oral administration, one or more active substances may be mixed with at least one inert diluent such as lactose, mannitol, glucose, microcrystalline cellulose, hydroxypropylcellulose, starch, polyvinyl pyrrolidone, or aluminum magnesium silicate. In the usual way, the composition may contain additives other than the inert diluent, such as a lubricant, a disintegrating agent, a stabilizing agent, or a solubilizing or solubilization assisting agent. If necessary, tablets or pills may be coated with a sugar coating or a film of a gastric or enteric substance.

The liquid composition for oral administration may include, for example, emulsions, solutions, suspensions, syrups, and elixirs, and may contain a generally used inert diluent such as purified water or ethyl alcohol. The composition may contain additives other than the inert diluent, such as moistening agents, suspending agents, sweeteners, flavors, or antiseptics.

The injections for parenteral administration may include aseptic aqueous or non-aqueous solutions, suspensions, and emulsions. Examples of the diluent for use in the aqueous solutions and suspensions include distilled water for injection use and physiological saline. Examples of the diluent for use in the non-aqueous solutions and suspensions include propylene glycol, polyethylene glycol, plant oil (e.g., olive oil), alcohols (e.g., ethanol), polysorbate 80 and the like. Such a composition may further contain a moistening agent, an emulsifying agent, a dispersing agent, a stabilizing agent, a solubilizing or solubilization assisting agent, an antiseptic or the like. These compositions may be sterilized, for example, by filtration through a bacteria retaining filter, blending of a germicide, or irradiation. Alternatively, these compositions may be used by first making them into sterile solid compositions and dissolving them in sterile water or other sterile solvent for injection use prior to their use.

The dose is optionally decided by taking into consideration the strength of each active ingredient used, symptoms, age, sex, or the like of each patient to be administered. For example, in an oral administration, the usual dosage for an adult (60 kg in weight) is about 0.1 to 5000 mg, preferably 0.1 to 500 mg per day. In a parenteral administration, the usual dosage is about 0.01 to 1000 mg, preferably 0.01 to 100 mg per day in the form of an injection.

### 2. Polynucleotide, recombinant vector, and transformant of the present invention

The present invention includes a polynucleotide consisting of the nucleotide sequence of SEQ ID NO: 1 or a partial sequence thereof; and a polynucleotide having a promoter activity of a PGC-1β gene, and consisting of a nucleotide sequence in which 1 to 10 nucleotides are deleted, substituted, and/or added in the nucleotide sequence of SEQ ID NO: 1 or a partial sequence thereof. The present invention includes a recombinant vector comprising at least one of the above polynucleotides, and a transformant comprising at least one of the above polynucleotides.

Whether or not a certain polynucleotide (hereinafter referred to as a test polynucleotide) exhibits a promoter activity of a PGC-1β gene may be confirmed by, for example, a method described in Example 10. More particularly, a reporter vector in which a test polynucleotide is linked to the upstream region of an appropriate reporter gene (for example, luciferase) is constructed, and appropriate host cells are transfected with the reporter vector. Whether or not the test polynucleotide exhibits the promoter activity of a PGC-1β gene may be confirmed by analyzing whether or not an expression of the reporter gene in the host cell is induced by a treatment of forskolin and dexamethasone. The expression of the reporter gene induced in the presence of forskolin and dexamethasone is preferably 1.5 times or more, more preferably 2 times or more, in comparison with that in the absence of forskolin and dexamethasone. As shown in Example 10, the polynucleotide consisting of the nucleotide sequence of SEQ ID NO: 1 is a human PGC-1β promoter having a promoter activity of a PGC-1β gene.

Those skilled in the art may prepare a DNA having a promoter activity similar to a naturally-occurring promoter activity, by performing modifications, such as deletion, substitution, and/or addition, in a part of a naturally-occurring promoter. The polynucleotide of the present invention includes a polynucleotide consisting of a nucleotide sequence in which one or more nucleotides are deleted, substituted, and/or added in a naturally-occurring nucleotide sequence and having a promoter activity similar to that of the polynucleotide consisting of the nucleotide of SEQ ID NO: 1 (i.e., the promoter activity of the PGC-1β gene). The number of nucleotides to be deleted, substituted, and/or added is preferably 1 to 10, more preferably 1 to 5.

The nucleotide modifications may be performed by, for example, an introduction of deletion by a restriction enzyme or DNA exonuclease, an introduction of variations by site-specific mutagenesis [Nucleic Acid Res. 10, 6487 (1982)], a modification of a promoter sequence by a PCR method using a primer for mutation, a direct introduction of a synthetic mutant DNA [Maniatis, T. et al. (1989) : Molecular Cloning - A Laboratory Manual 2nd Edt. Cold Spring Harbor Laboratory, NY], or the like.

The recombinant vector and transformant of the present invention may be prepared by conventional methods using the polynucleotide of the present invention. Host cells commonly used in genetic engineering may be used as a host cell for preparing the transformant of the present invention. As the host cells, there may be mentioned, for example, host cells derived from eukaryotes, such as vertebrates (preferably mammals), insects, or yeast, or host cells derived from prokaryotes, such as Escherichia coli. As an expression vector for preparing the recombinant vector of the present invention, an appropriate vector may be selected in accordance with the host cell used.

### 3. Screening method of the present invention

The present invention includes a method of screening for an activating agent of glucose uptake, comprising the steps of:
bringing a substance to be tested into contact with a polynucleotide (hereinafter referred to as a polynucleotide for screening) comprising the polynucleotide of the present invention; and
analyzing (preferably measuring) a promoter activity of a PGC-1β gene.

According to a preferred embodiment of the screening method of the present invention, the polynucleotide for screening contains a reporter gene at the downstream region thereof, and the step of analyzing a promoter activity of a PGC-1β gene is a step of analyzing an expression of the reporter gene. As the reporter gene, there may be mentioned, for example, a luciferase gene or a β-galactosidase gene, and a luciferase gene is most preferable.

The "step of bringing a substance to be tested into contact with a polynucleotide for screening" is not particularly limited, so long as a test substance may be brought into contact with the polynucleotide, and a method described in Example 11 is preferable. More particularly, the contacting step may be performed by constructing a reporter vector in which the polynucleotide for screening is linked to the upstream region of an appropriate reporter gene (for example, luciferase), transfecting an appropriate host cell with the reporter vector, and cultivating the transfected host cells for a predetermined time (for example, 24 hours) in the presence of a test substance.

The "step of analyzing a promoter activity of a PGC-1β gene" is not particularly limited, so long as a promoter activity of a PGC-1β gene may be analyzed, and a method described in Example 11 is preferable. More particularly, after the contacting step, the analyzing step may be performed by analyzing reporter activities in the cells in the presence and absence of a test substance by an assay selected in accordance with the reporter gene used, and comparing the results.

The test substances which may be used in the screening method of the present invention include, for example, compounds (including proteins and DNAs) and mixtures (for example, compositions, extracts, or cultures). The test substances include artificially synthesized substances and isolated naturally-occurring substances, and may be low molecular weight compounds or high molecular weight compounds, or organic substances or inorganic substances.

Hereinafter, an embodiment of the screening method of the present invention will be explained.
Test substances are brought into contact with cells (preferably animal cells or yeast) containing a reporter gene linked to the downstream region of the promoter gene of the present invention, to select a substance capable of increasing an expression of the reporter gene. For example, as shown in Example 11, a vector in which a reporter gene (for example, luciferase) is linked to the downstream region of the promoter of the present invention is constructed, and host cells (for example, yeast or animal cells) are transfected with the vector, to prepare cells stably or transiently expressing the promoter-reporter gene. Test substances are brought into contact with the cells to select a substance capable of inducing an expression of the reporter gene. A substance capable of activating a promoter of a PGC-1β gene can be selected by the above method, and an activating agent of glucose uptake can be obtained.

As a concrete screening method, a method described in Example 11 is preferable. As substances capable of activating the promoter, it is preferable to select a substance in which the activity of activating the promoter is 2 times or more, in comparison with a control case where the substance is absent.

In this connection, whether or not a substance obtained by the screening method has a function of activating glucose uptake may be confirmed by a conventional method known to those skilled in the art, or a modification thereof, such as a method described in Example 6. More particularly, appropriate cells [preferably muscle cells, such as a rat myoblast L6 (ATCC, CRL-1458) or a mouse myoblast C2C12 (ATCC, CRL-1772)] may be cultivated for a predetermined time (for example, 10 minutes) in the presence of labeled glucose, and an amount of glucose incorporated into the cells may be measured by using the label as an index, to confirm the function of activating glucose uptake.

### 4. Analyzing method of the present invention

The present invention includes a method of analyzing an activity of glucose uptake, comprising the steps of:
bringing a substance to be tested into contact with the polynucleotide for screening,
analyzing (preferably measuring) a promoter activity of a PGC-1β gene, and
analyzing (preferably measuring) an activity of glucose uptake caused by the test substance.

The "step of bringing a substance to be tested into contact with the polynucleotide for screening" and the "step of analyzing a promoter activity of a PGC-1β gene" may be performed in accordance with a method similar to that described in "3. Screening method of the present invention". The "step of analyzing an activity of glucose uptake caused by the test substance" may be performed in accordance with a method similar to that for confirming an activity of activating glucose uptake, described in "3. Screening method of the present invention".

### EXAMPLES

The present invention now will be further illustrated by, but is by no means limited to, the following Examples. In this connection, the following procedures may be performed in accordance with known methods [for example, Maniatis, T. et al. (1989) : Molecular Cloning - A Laboratory Manual 2nd Edt. Cold Spring Harbor Laboratory, NY], unless otherwise specified.

### Example 1: Cell cultivation

A tissue into which the largest amount of glucose can be incorporated in a living body is muscle. In this example and the following examples, a rat myoblast L6 was used to detect an activity of glucose uptake in vitro. L6 cells (ATCC, CRL-1458) were maintained in a Dulbecco's modified Eagle's medium (GIBCO BRL, USA, 11995-065) supplemented with 10% fetal bovine serum (JRH BIOSCIENCES, Cat. No. 12303-500M), and cultured in a humid atmosphere containing 5% CO₂ at 37°C. When the L6 cells were confluent, the medium was changed to a Dulbecco's modified Eagle's medium supplemented with 2% horse serum (GIBCO, Cat. No. 16050), and further cultured for 5 days to perform an induction of differentiation.

### Example 2: Cloning of human PGC-1β gene

An oligonucleotide (SEQ ID NO: 2) consisting of 25 nucleotides of the N-terminal sense sequence of a human PGC-1β gene (PERC, GenBank Accession No. NM_133263) and an oligonucleotide (SEQ ID NO: 3) consisting of 29 nucleotides of the C-terminal antisense sequence thereof were synthesized, and used as PCR primers. A cDNA library from human skeletal muscle (cDNA Library Human Skeletal Muscle, TaKaRa, Code No.9514) was used as a template, and a PCR was carried out using an enzyme for PCR, Pfu DNA polymerase (Stratagene, Cat No.600135), in accordance with a manual attached thereto, to obtain a DNA fragment of approximately 3 kb containing the human PGC-1β gene.

### Example 3: Construction of recombinant adenovirus for overexpressing human PGC-1β

The DNA fragment of approximately 3 kb containing the human PGC-1β gene, which was obtained in Example 2, was inserted into the KpnI-NotI site of a shuttle vector (pAdTrack-CMV) for adenovirus, to obtain a shuttle vector (pAdTrack-hPGC-1β) for adenovirus overexpressing human PGC-1β. The obtained pAdTrack-hPGC-1β was linearized by a digestion with PmeI, and Escherichia coli BJ5183 was cotransformed with the linearized vector and an adenovirus backbone vector pAdEasy-1, to obtain a vector (pAdEasy-hPGC-1β) for adenovirus overexpressing human PGC-1β by homologous recombination. The obtained pAdEasy-hPGC-1β was linearized by a digestion with PacI, and 293 cells were transfected with the linearized vector using a reagent for transfection (FuGENE^{™}6, Roche, Cat. No. 1815091) in accordance with a manual attached thereto. A recombinant adenovirus (Ad-hPGC-1β) for expressing human PGC-1β was obtained from the 293 cells in accordance with conventional methods. In this connection, the shuttle vector pAdTrack-CMV, backbone vector pAdEasy-1, Escherichia coli BJ5183, and 293 cells contained in an Adeasy System (Johns Hopkins University) were used as the above-mentioned vectors and cells.

### Example 4: Confirmation of induction of human PGC-1β expression in L6 by Ad-hPGC-1β infection

In accordance with the procedures described in Example 1, differentiated L6 cells were prepared in 24-well collagen coated dishes (Asahi Glass Cat. No. 4820-010). The cells were infected with the recombinant adenovirus Ad-hPGC-1β (5x10⁹ pfu/well), and further cultured for 24 hours. In addition, a recombinant adenovirus (Ad-GFP) overexpressing only GFP was used as a control, and a recombinant adenovirus (Ad-hPGC-1α) overexpressing human PGC-1α was used for comparison. In this connection, Ad-hPGC-1β and Ad-hPGC-1α express GFP in the same system as that of Ad-GFP to confirm an infection rate.

After 24 hours from the infection, each cell group was lysed in 100 µL of a sample buffer (EzApply, ATTO, Cat. No. 2332330) for SDS-polyacrylamide gel electrophoresis (SDS-PAGE). Samples were separated by SDS-PAGE (10% polyacrylamide), and transferred to a membrane (Immobilon, MILLIPORE, Cat. No. IPVH00010) using a semi-dry transfer cell [TRANS-BLOT (registered trademark) SD SEMI-DRY TRANSFER CELL, BIO-RAD]. The membrane was incubated in a TBS-T (Tris-HC1 pH7.4, 15 mmol/L NaCl, and 0.005% Tween20) containing 5% skimmed milk at room temperature for 2 hours to perform blocking. The membrane was reacted with an antihuman-PGC-1β antibody (Immuno-Biological laboratories) diluted to 1/500 with TBS-T containing 5% skimmed milk at 4°C for 10 hours, and human PGC-1β in each sample was detected by chemiluminescence (ECL Plus Western Blotting Detection Reagents, Amersham, Cat. No, RPN2132) caused by horseradish peroxidase (HRP).

As a result, a band of human PGC-1β specific to the cell group infected with Ad-hPGC-1β was detected, and an overexpression of human PGC-1β in the differentiated L6 cells was confirmed. In this connection, an amount of gene expressed was measured 4 times per group, and each experiment was repeated at least twice, to confirm the reproducibility.

### Example 5: Induction of gene expression of glucose transporter GLUT4 in muscle cells overexpressing human PGC-1β

The cells overexpressing human PGC-1β, prepared in accordance with the procedures described in Example 4, were used to prepare total RNAs therefrom by using a commercially available reagent [RNeasy (registered trademark) Micro Kit, QIAGEN, Cat. No. 74004] in accordance with a manual attached thereto. The RNAs were used as a template to prepare cDNAs by using a commercially available reagent (SuperScript^{™} III RNaseH- Reverse Transcriptase, Invitrogen, Cat. No. 18080-044) in accordance with a manual attached thereto. The cDNAs were used as a template to quantify an amount of each cDNA by real-time PCR (7900HT Sequence Detection System, Applied Biosystems) in accordance with a manual attached thereto. Primers of sense and antisense strands of each gene and a commercially available reagent [SYBRy (registered trademark) GREEN PCR Master Mix, Applied Biosystems, Cat. No. 4309255] were used for the quantification. The quantified genes were β-actin and GLUT4. The nucleotide sequences of the sense and antisense strand primers of β-actin were those of SEQ ID NOS: 4 and 5, respectively. The nucleotide sequences of the sense and antisense strand primers of GLUT4 were those of SEQ ID NOS: 6 and 7, respectively.

An amount of GLUT4 expressed was corrected with that of β-actin expressed of the same sample, and shown as a relative value when a corrected amount in a control (Ad-GFP-infected cell group) is regarded as 1 (Figure 1). As a result, it was first clarified that an expression of the endogenous GLUT4 gene in the L6 cell was significantly induced by the overexpression of human PGC-1β. This result suggested a possibility that the overexpression of human PGC-1β would activate glucose uptake on the basis of an induction of GLUT4 expression in muscle cells. In this connection, an amount of gene expressed was measured 4 times per group, and each experiment was repeated at least twice, to confirm the reproducibility. In addition, an amount of β-actin gene expressed did not change.

### Example 6: Measurement of activity of glucose uptake in muscle cells overexpressing human PGC-1α/β

The cells overexpressing human PGC-1β or human PGC-1α, prepared in accordance with the procedures described in Example 4, were used to measure an activity of glucose uptake. After 48 hours from the infection, the cells were rinsed with 1 mL of a KRP buffer (136 mmol/L KC1, 4.7 mmol/L KC1, 1.25 mmol/L CaCl₂·2H₂O, 1.25 mmol/L MgSO₄·7H₂O, and 5 mmol/L Na₂HPO₄·12H₂O), and further cultured in 500 µL of KRP for 1 hour. The buffer was changed to KRP supplemented with 100 nmol/L insulin, and the cells were further cultured for 20 minutes. The buffer was changed to KRP supplemented with 1 mmol/L 2-deoxy-glucose and a radiolabeled compound thereof, 10 kBq 2-deoxy-D-[U-¹⁴C] glucose (Amersham Bioscience, Cat. No. CFB195), and the cells were further cultured for 10 minutes. The cells were washed with an ice-cold PBS (phosphate-buffered saline) buffer 3 times, and lysed in 200 µL of 0.1% SDS. Each lysate was mixed with 2 mL of a liquid scintillator (AQUASOL 2, PERKINELMER, Cat. No. 6NE9529), and an amount of radiolabeled compound (radioactivity, CPM) incorporated into the cells was measured using a liquid scintillation counter (PACKARD, B2500TR).

The amounts of radiolabeled compound (CPM) incorporated into the cells are shown in Figure 2. It was first clarified that the infection of Ad-hPGC-1β, i.e., the overexpression of human PGC-1β, significantly induced the activity of glucose uptake in the differentiated L6 cells. Further, it was found that the cells overexpressing human PGC-1β showed the activity of glucose uptake much more highly than those overexpressing human PGC-1α, and exhibited the promoting activity even in the absence of insulin, in which the cells overexpressing human PGC-1α did not significantly exhibit the promoting activity. In this connection, an amount of radiolabeled compound was measured 4 times per group, and each experiment was repeated at least twice, to confirm the reproducibility.

### Example 7: Cloning of promoter region of human PGC-1β gene

In view of the human PGC-1β gene (PERC, GenBank Accession No. NM_133263.1) and a public human genomic database (GenBank nucleotide database), a sense oligonucleotide (SEQ ID NO: 8) consisting of 29 nucleotides and an antisense oligonucleotide (SEQ ID NO: 9) consisting of 30 nucleotides were synthesized, and used as PCR primers to obtain a DNA fragment of approximately 1 kb upstream of the human PGC-1β gene. The promoter region was cloned by using a human genomic DNA (Human Genomic DNA, CLONTECH, Cat. No. 6550-1) as a template and a Pfu DNA polymerase (Stratagene, Cat No.600135) as an enzyme, in accordance with manuals attached thereto. As a result, a DNA fragment of approximately 1 kb was obtained.

### Example 8: Sequence analysis of promoter of human PGC-1β gene

The DNA fragment obtained in Example 7 was subcloned into a pCR (registered trademark) 2.1-TOPO vector by using a commercially available kit [TOPO^{™} TA Cloning (registered trademark) Kit, Invitrogen (registered trademark), USA, IV450002] in accordance with a manual attached thereto, to designate a subclone containing the DNA fragment of approximately 1 kb as pCR-hPGCβ(1.0). The nucleotide sequence of the subcloned DNA was determined using a commercially available kit [BigDye^{™} Terminator Cycle Sequencing Kit, ABI PRISM, PE APPLIED Biosystems, USA, 4303125] and a DNA sequencer (ABI PRISM^{™} 377 DNA Sequencer). The nucleotide sequence of the cloned DNA fragment of approximately 1 kb is shown as that of SEQ ID NO: 1.

### Example 9: construction of reporter vector containing promoter of human PGC-1β gene

The pCR-hPGCβ(1.0) vector prepared in Example 8 was digested with restriction enzymes KpnI and MluI, and the obtained KpnI-MluI fragment containing the 1-kb upstream region of human PGC-1β gene was inserted into the KpnI-MluI site of a PicaGene basic vector 2 (Toyo Ink, Japan), to construct a human PGC-1β reporter vector designated as phPGC1β(1.0)Luc.

### Example 10: Measurement of activity of human PGC-1β promoter

L6 cells were transiently transfected with the phPGC1β(1.0)Luc plasmid constructed in Example 9, in accordance with the following procedures. L6 cells were cultivated in a tissue culture dish having a diameter of 10 cm to become 80% confluent. The cells were cotransfected with 5 µg of the phPGC1β(1.0)Luc plasmid and 1 µg of plasmid pCH110 (Pharmacia Biotech) using a regent for transfection (FuGENE^{™}6, Roche, Cat. No. 1815091). In this connection, the plasmid pCH110 containing a β-galactosidase gene regulated by a β-actin promoter was used to standardize an efficiency of transfection. After 24 hours from the transfection, the cells were transferred to a 96-well tissue culture dish, and further cultured for 6 hours. Since it was reported that the combination of forskolin and dexamethasone induced an expression of endogenous PGC-1β in hepatocytes [J. Biol. Chem., 278: 30843 (2003)], the cells were treated with 10 µmol/L (final concentration) forskolin and 1 µmol/L dexamethasone [solvent = dimethyl sulfoxide (DMSO)] for 24 hours. After the treatment, the cells were lysed with a solution for cell lysis (LCβ; Toyo Ink, Japan), and a luciferase activity therein was measured using a PicaGene luminescence kit (Toyo Ink, Japan, 309-04321). In this connection, the activity was measured 3 times per group, and each experiment was repeated at least twice, to confirm the reproducibility.

The result is shown in Figure 3. Each reporter activity was standardized using the β-galactosidase activity, and shown as a relative value when the value in a control (not treated with the compounds, i.e., DMSO as the solvent was added alone) is regarded as 1. The human PGC-1β reporter was increased 1.6 times at the maximum by the treatment of forskolin and dexamethasone in the L6 cells. The result accorded with the report that the combination induced the expression of the endogenous gene 2 times or more in hepatocytes, and it was confirmed that the polynucleotide consisting of the nucleotide sequence of SEQ ID NO: 1 was a human PGC-1β promoter or a polynucleotide containing the same.

### Example 11: Screening for activating agent of human PGC-1β promoter

In accordance with the procedures described in Example 10, commercially available compounds were screened using the reporter vector phPGC1β(1.0)Luc, to identify a compound capable of inducing a promoter activity of human PGC-1β. As a result, 2-(3-aminophenyl)-5-benzoyl-(1H)-benzimidazole] (ZELINSKY, Cat. No. A1773/0075171) (hereinafter referred to as compound A) was obtained, as a compound in which the activating activity of the promoter is 2 times or more at a final concentration of 1 µmol/L to 10 µmol/L, in comparison with that in the absence of the compound. Reporter activities (luciferase activities) when compound A was added and that when no compound was added are shown as relative values in Figure 4.

### Example 12: Measurement of activity of compound A in glucose uptake

An effect of the compound A on the activity of glucose uptake was examined in accordance with the procedures described in Example 6, using differentiated L6 cells prepared by the procedures described in Example 1. The compound A was obtained in Example 11, and exhibited the activating activity of a human PGC-1β promoter. The differentiated cells were cultured in a differentiation-inducing medium supplemented with the compound A at various final concentrations of 1 µmol/L to 10 µmol/L for 24 hours, and the activity of glucose uptake in the cells was measured. As a result, it was clarified that the compound A significantly activated glucose uptake in muscle cells, as shown in Figure 5.

### INDUSTRIAL APPLICABILITY

The activating agent of glucose uptake of the present invention, or activating agents of glucose uptake obtained by the method of the present invention may be applied to the treatment and/or prevention of diabetes.
Although the present invention has been described with reference to specific embodiments, various changes and modifications obvious to those skilled in the art are possible without departing from the scope of the appended claims.

## Claims

1. An activating agent of glucose uptake comprising as an active ingredient a substance having a PGC-1β function.

2. A method for activating glucose uptake, comprising administering to a subject in need thereof a substance having a PGC-1β function, in an amount effective therefor.

3. Use of a substance having a PGC-1β function in the manufacture of an activating agent of glucose uptake.

4. A polynucleotide selected from the group consisting of:
a polynucleotide consisting of a nucleotide sequence in which 1 to 10 nucleotides are deleted, substituted, and/or added in the nucleotide sequence of SEQ ID NO: 1 or a partial sequence thereof, and having a promoter activity of a PGC-1β gene; and
a polynucleotide consisting of the nucleotide sequence of SEQ ID NO: 1 or a partial sequence thereof.

5. The polynucleotide according to claim 4, consisting of a nucleotide sequence in which 1 to 10 nucleotides are deleted, substituted, and/or added in the nucleotide sequence of SEQ ID NO: 1, and having a promoter activity of a PGC-1β gene; or consisting of the nucleotide sequence of SEQ ID NO: 1.

6. Use of the polynucleotide according to claim 4 as a promoter of a gene.

7. A recombinant vector, comprising the polynucleotide according to claim 4.

8. A transformant comprising the polynucleotide according to claim 4.

9. Use of the polynucleotide according to claim 4 as a screening tool for an activating agent of glucose uptake.

10. Use of the polynucleotide according to claim 4 in the screening for an activating agent of glucose uptake.

11. A method of screening for an activating agent of glucose uptake, comprising the steps of:
bringing a substance to be tested into contact with a polynucleotide comprising the polynucleotide according to claim 4; and
analyzing a promoter activity of a PGC-1β gene.

12. The method according to claim 11, wherein the polynucleotide comprising the polynucleotide according to claim 4 contains a reporter gene at the downstream region thereof, and the step of analyzing a promoter activity of a PGC-1β gene is a step of analyzing an expression of the reporter gene.

13. A method of analyzing an activity of glucose uptake, comprising the steps of:
bringing a substance to be tested into contact with the polynucleotide comprising the polynucleotide according to claim 4,
analyzing a promoter activity of a PGC-1β gene, and
analyzing an activity of glucose uptake caused by the test substance.
